Europäisches Patentamt

European Patent Office

Office européen des brevets

(19)

(11) Veröffentlichungsnummer: **0 091 060**
**B1**

(12) **EUROPÄISCHE PATENTSCHRIFT**

(45) Veröffentlichungstag der Patentschrift: **20.01.88**

(21) Anmeldenummer: **83103060.6**

(22) Anmeldetag: **28.03.83**

(51) Int. Cl.⁴: **C 07 C 47/055,** C 07 C 45/38,
B 01 J 23/50, B 01 J 23/72,
B 01 J 23/89, B 01 J 27/18,
B 01 J 21/08, B 01 J 21/06

(54) **Verfahren zur Herstellung von Formaldehyd.**

(30) Priorität: **08.04.82 DE 3213227**

(43) Veröffentlichungstag der Anmeldung:
**12.10.83 Patentblatt 83/41**

(45) Bekanntmachung des Hinweises auf die
Patenterteilung:
**20.01.88 Patentblatt 88/03**

(84) Benannte Vertragsstaaten:
**BE CH DE FR GB LI**

(56) Entgegenhaltungen:
EP-A-0 072 912
DE-A-2 322 757
DE-B-1 231 229
DE-B-2 520 219
DE-C-1 285 995
DE-C-1 294 360
GB-A-1 272 592
US-A-2 656 322

B.I.O.S. FINAL REPORT, Nr. 978, Seiten 2-6;
"Manufacture of formaldehyde"

F.I.A.T. FINAL REPORT, Nr. 999, Seiten 1-3;
"Formaldehyde manufacture in the I.G.
Farbenindustrie"

(73) Patentinhaber: **BASF Aktiengesellschaft**
**Carl-Bosch-Strasse 38**
**D-6700 Ludwigshafen (DE)**

(72) Erfinder: **Reuss, Guenter, Dr.**
**Theaterplatz 10**
**D-6700 Ludwigshafen (DE)**
Erfinder: **Petri, Norbert, Dr.**
**Max-Beckmann-Vertrasse 17**
**D-6710 Frankenthal (DE)**
Erfinder: **Lehmann, Gunter**
**Schlesier Strasse 4**
**D-6701 Birkenheide (DE)**
Erfinder: **Pitteroff, Walter, Dr.**
**In den Hahndornen 10**
**D-6719 Bobenheim (DE)**

Courier Press, Leamington Spa, England.

EP 0 091 060 B1

0 091 060

**Beschreibung**

Die Erfindung betrifft ein Verfahren zur Herstellung von Formaldehyd durch oxidierende Dehydrierung von Methanol in Gegenwart eines Katalysators mit einer Gesamtschichtdicke von 5 bis 50 Millimeter, der aus 3 oder mehr Schichten besteht, wobei ein Teil der Schichten 5 bis 45 Gew.% des Katalysators mit Silberkristallen der Korngröße 0,2 bis 1,75 Millimeter, ein Teil der Schichten 1 bis 35 Gew.% des Katalysators mit Titanmonoxid- und/oder Quarzteilchen der Korngröße 0,1 bis 2,5 Millimeter und der restliche Teil der Schichten 20 bis 94 Gew.% des Katalysators mit Teilchen von 99 bis 99,9999 Gew.% Kupfer und/oder Silber und 0,0001 bis 1 Gew.% Phosphor, bezogen auf das Gewicht dieser Teilchen an diesen Elementen, und der Korngröße 0,75 bis 2,5 Millimeter enthalten.

In Ullmanns Encyklopädie der technischen Chemie, Band 7, Seiten 659 ff, sind verschiedene Verfahren zur Herstellung von Formaldehyd durch oxidierende Dehydierung von Methanol in Gegenwart eines Silberkatalysators bei erhöhter Temperatur beschrieben. Im FIAT-Bericht Nr. 999, Seiten 1 bis 3, wird eine Anordnung des Silberkatalysators in einer Schicht mit der Schichtdicke von 10 bis 15 Millimeter beschrieben. Körner mit Korngrößen zwischen 1,25 Millimeter und 0,15 Millimeter Durchmesser werden in einer einzigen Schicht verwendet, wobei die gröberen Körner unten liegen. Die normale Lebensdauer des Katalysators beträgt 6 Monate. Wenn jedoch Methanol von hoher Permanganat-Reaktivität verwendet wird, kann die Lebensdauer auf 2 Monate oder weniger absinken. Der Umsatz des Methanols beträgt ca. 94%, die Ausbeute an Formaldehyd 82,5% der Theorie.

Im BIOS-Bericht Nr. 978, Seiten 2 bis 6, wird ein 4-Schichtenkatalysator folgender Zusammensetzung beschrieben, wobei sich die gröberen Teilchen am Boden der Schicht befinden:

| Korngröße mm | Gew.% des gesamten Katalysators |
|---|---|
| 0,15 | 8 |
| 0,32 | 44 |
| 0,64 | 36 |
| 1,23 | 12 |

Die Lebensdauer des Katalysators hängt von der Methanolqualität ab. Man verwendet ein gereinigtes Methanol; das Rohmethanol wird mit Permanganat behandelt und anschließend destilliert. Der Bericht erwähnt, daß der Katalysator üblicherweise 6 Monate Lebensdauer hat. In Übereinstimmung damit lehrt vorgenannter FIAT-Bericht, daß die Lebensdauer auf 2 Monate oder weniger absinken kann, wenn Methanol von hoher Permangant-Reaktivität, d. h. Rohmethanol, verwendet wird. Im allgemeinen erhält man eine Ausbeute von 82,2% der Theorie.

In der deutschen Patentschrift 1 231 229 wird ein in zwei Schichten angeordneter Katalysator beschrieben, wobei die untere Schicht aus mindestens 50 Gew.% Kristallen der Korngröße 1,25 bis 5 Millimeter besteht. Als obere Schicht werden Kristalle von 0,2 bis 1 Millimeter verwendet. Die Höhe der unteren Schicht wählt man zwischen 15 und 50 Millimeter, Als Höhe der oberen Schicht ist im Beispiel 2 1 bis 2 Millimeter angegeben. Die Lebensdauer des Zweischichtenkatalysators wird in Beispiel 2 mit 91 Tagen, die Ausbeute mit 88,3% der Theorie angegeben.

Bei der in der deutschen Auslegeschrift 12 94 360 beschriebenen Verfahrensweise wird ein Zweischichtenkatalysator verwendet, dessen untere Schicht zumindest 50 Gew.% aus Kristallen der Korngrößen 1 bis 4 Millimeter und dessen obere Schicht aus Kristallen mit Korngrößen von 0,1 bis 0,9 Millimeter besteht. Die Schichtdicke der unteren Schicht ist 15 bis 40 Millimeter, die der oberen Schicht, 0,75 bis 3 Millimeter. Wie die Beispiele zeigen, verwendet man als untere Schicht 94 Gew.% des Gesamtkatalysators mit Korngrößen von 1 bis 3 Millimeter und erzielt eine Lebensdauer des Katalysators von 70 Tagen und eine Ausbeute von 89%.

In der deutschen Patentschrift 1 285 995 wird ein Verfahren beschrieben, bei dem ein Silberkatalysator verwendet wird, der zu 7/8 seines Gewichtes aus Silberkristallen von 0,75 bis 3 Millimeter Durchmesser und zu 1/8 aus Silberkristallen unter 0,3 Millimeter zusammengesetzt ist und man sehr rasch die Reaktionsgase so abkühlt, daß der Kohlenmonoxidgehalt des Abgases nicht über 0,23 Volumenprozent ansteigt.

Es ist aus der DE—A—23 22 757 bekannt, daß man Formaldehyd durch oxidierende Dehydrierung von Methanol in Gegenwart eines Silberkatalysators mit einem Katalysator mit der Gesamtschichtdicke von 15 bis 35 Millimeter und 3 oder mehr Schichten Silberkristallen herstellt, wobei ein Teil der Schichten 72,5 bis 89 Gew.% des Katalysators mit Teilchen der Korngröße 1 bis 2,5 Millimeter, ein Teil der Schichten 2,5 bis 7,5 Gew.% des Katalysators mit Teilchen der Korngröße 0,75 bis 1 Millimeter und der restliche Teil der Schichten 8,5 bis 20 Gew.% des Katalysators mit Teilchen der Korngröße 0,2 bis 0,75 Millimeter enthalten. Man erhält Ausbeuten bis zu 88% der Theorie. Auch Rohmethanol kann verwendet werden. Die Lebensdauer des Katalysators beträgt in der Regel mindestens 100 Tage im Falle von Rohmethanol.

2

In der US—A—2 656 322 wird ein Katalysator beschrieben, der im wesentlichen aus mindestens einem Metall der Gruppe Al, Cd, Zn, Cu besteht und mit einem Feststoff bedeckt ist, der durch Kalzinieren einer Mischung aus z.B. Phosphorsäure und einem Reaktionsprodukt aus vorgenanntem Metall und der vorgenannten Säure, entstanden ist. In der Beschreibung wird angegeben, daß die erfindungsgemäßen Katalysatoren für die Dehydrofluorierung (Spalte 4, 11 und 12), Polymerisation ungesättigter Kohlenwasserstoffe, Alkylierung aromatischer Kohlenwasserstoffe, Dehydrierung von Alkoholen zu Olefinen, vorzugsweise mit 2 bis 5 C-Atomen, Hydrierung von Olefinen zu Alkoholen und Oxidation von Methan zu Methanol und Formaldehyd, geeignet sind. Zahlreiche Metalle, auch Silber, können verwendet werden (Spalte 4, Zeilen 30 bis 58). Der Katalysator wird durch Eintauchen des Metalls in Orthophosphorsäure behandelt und vorzugsweise anschließend auf 260 bis 371°C erwärmt (Spalte 5, Zeilen 5 bis 15). Die Phosphorsäure ist mindestens 75-, bevorzugt 85-gewichtsprozentig (Spalte 4, Zeilen 72 bis 75). Die Katalysator-Partikelgröße ist im allgemeinen 1,35 bis 4 Millimeter (Spalte 5, Zeile 22). Die Metalle, die in der Spannungsreihe unter dem Wasserstoff stehen, reagieren nicht mit der Säure, sondern müssen erst oxidiert werden. Es wird beschrieben, daß Silber in Gegenwart eines Peroxides mit Sauerstoff behandelt oder mit Schwefelwasserstoff in Silbersulfid und anschließend mit Sauerstoff in das Oxid überführt wird (Spalte 5, Zeilen 26 bis 60). In den Beispielen werden nur Dehydrofluorierungen beschrieben.

In der GB—A—1 272 592 wird ein Verfahren zur oxidativen Umsetzung von Alkoholen mit einer Sauerstoff enthaltenden Gasmischung zu Carbonylverbindungen in der Gasphase bei erhöhter Temperatur an einem Katalysator, der als wirksame Substanz Kupfer und/oder Silber sowie Phosphor als Promoter enthält, beschrieben. In allen Beispielen werden lediglich Diole umgesetzt. In der Beschreibung wird darauf hingewiesen, daß der Prozeß besonders für die Oxidation von Diolen zu den entsprechenden Carbonylverbindungen verwendet werden kann (Seite 2, Zeile 78). Eine Reaktionstemperatur von 180 bis 600°C, vorzugsweise 300 bis 500°C, wird empfohlen (Seite 2, Zeilen 63 bis 65). Der Prozeß ist anwendbar auf Alkanole mit 1 bis 6 Kohlenstoffatomen (Seite 2, Zeilen 33 bis 41). Eine bevorzugte Ausführungsform ist eine Legierung, wobei unter Legierung auch eine Lösung diskreter Teile im Metall oder eine Mischung verschiedener diskreter Legierungsphasen gemeint sein kann. Phosphor befindet sich im Katalysator als Legierungskomponente.

In der US—A—2 005 645 wird die Umsetzung niedriger aliphatischer Alkohole mit einer Sauerstoff enthaltenden Gasmischung an einem Silberkatalysator, der durch Reduktion von Silberoxid gewonnen wurde, beschrieben. Als Promotoren werden noch die Oxide von V, Ce, Th, Al, Cr, Zn, Mo und W angegeben. Die Umsetzung von Methanol zu Formaldehyd in Gegenwart von Silber und gegebenenfalls diesen Promotoren in einer einzigen Schicht wird gezeigt. Die Ausbeuten an Formaldehyd, bezogen auf verwendetes Methanol, betragen 71 bis 81 Prozent.

Es wurde nun gefunden, daß man Formaldehyd durch oxidierende Dehydrierung von Methanol in Gegenwart eines Silberkatalysators mit mehreren Schichten von Katalysatorteilchen bei erhöhter Temperatur vorteilhaft erhält, wenn die Umsetzung mit einem Katalysator mit 3 oder mehr Schichten Katalysatorteilchen und einer Gesamtschichtdicke von 5 bis 50 Millimeter durchgeführt wird, wobei

a) ein Teil der Schichten 5 bis 45 Gew.% des Katalysators mit Silberkristallen der Korngröße 0,2 bis 1,75 Millimeter,

b) ein Teil der Schichten 1 bis 35 Gew.% des Katalysators mit Titanmonoxid- und/oder Quarzteilchen der Korngröße 0,1 bis 2,5 Millimeter und

c) der restliche Teil der Schichten 20 bis 94 Gew.% des Katalysators mit Teilchen von 99 bis 99,9999 Gew.% Kupfer und/oder Silber und 0,0001 bis 1 Gew.% Phosphor, bezogen auf das Gewicht dieser Teilchen an diesen Elementen, und der Korngröße 0,75 bis 2,5 Millimeter enthalten.

Im Vergleich zu den im Stand der Technik aufgeführten Verfahren liefert das Verfahren nach der Erfindung überraschend auf einfachem und wirtschaftlichem Wege Formaldehyd in einem besseren Gesamtergebnis mit Bezug auf Ausbeute, Raum-Zeit-Ausbeute und Reinheit des Endstoffs sowie Lebensdauer des Katalysators. Auch Rohmethanol kann verwendet werden. Die Lebensdauer des Katalysators beträgt in der Regel mindestens 100 Tage im Falle von Rohmethanol. Alle diese vorteilhaften Ergebnisse sind im Hinblick auf den Stand der Technik, insbesondere auf die Lehre der 4 letztgenannten Patentschriften, überraschend, denn man hätte die Notwendigkeit der 3 erfindungsgemäßen Katalysatorkomponenten, insbesondere der TiO- und/oder Quarzteilchen, mit ihren Teilchen bestimmter Korngröße, bestimmter Schichtdicke und Gewichtsanteilen nicht erwartet. Weiterhin ist überraschend, daß 3 oder mehr Schichten dieser Komponenten und die Herstellung der phosphorhaltigen Schicht durch eine einfache Behandlung mit Phosphorsäure ohne einen komplizierten Oxidationsvorgang vorteilhafte Ergebnisse liefert. Auch konnte im Hinblick auf GB—A—1 272 592 nicht erwartet werden, daß Phosphor nicht in den Katalysator eingebaut, sondern in dem erfindungsgemäßen Verfahren auf der Oberfläche abgelagert ist.

Für das Verfahren geeignete Ausgangsstoffe sind reines Methanol, technisches Methanol, nach einem Hochdruck- oder Niederdruck-Verfahren hergestelltes Rohmethanol oder vorteilhaft deren Mischungen mit Wasser; die Konzentration der wäßrigen Gemische liegt in der Regel bei 50 bis 98 Gew.%, vorzugsweise zwischen 60 und 95 Gew.% Methanol. In einer vorteilhaften Ausführungsform wird technisches Methanol, das nach den in DE—B—12 77 834, DE-Patentschrift 1 235 881 und DE-Patentschrift 1 136 318 beschriebenen Verfahren durch Reinigung in Gestalt der Abtrennung einer niedriger siedenden Fraktion bzw. der Behandlung mit Oxidationsmitteln und/oder Alkalien aus Rohmethanol erhalten werden kann, verwendet.

Das Methanol der Rohmethanol wird in Dampfform, vorteilhaft im Gemisch mit Wasserdampf und gegebenenfalls mit Inertgas, dem Reaktionsraum zugeführt. Als Inertgas kommt für das Verfahren beispielsweise Stickstoff in Betracht.

Als oxidierendes Agens lassen sich sowohl der reine Sauerstoff als auch freien Sauerstoff enthaltende Gase, insbesondere Luft, verwenden. Sauerstoff, in der Regel in Gestalt von Luft, und Methanol werden zweckmäßig im Molverhältnis von 0,25 bis 0,6, insbesondere von 0,35 bis 0,5 Mol Sauerstoff je Mol Methanol angewandt. Vorzugsweise beträgt die Gesamtmenge an Wasserdampf nicht mehr als 3, vorteilhaft von 0,67 bis 1,75 Mol je Mol Methanol.

Die Katalysatorteilchen befinden sich im Katalysator des üblicherweise vertikal aufgestellten Reaktors je nach Art, Gewichtsanteil und Korngröße in einem oberen, mittleren oder unteren Teil der Gesamtschicht (a+b+c) angeordnet. Ag/P und Cu/P können auch als Gemisch, vorzugsweise im Gewichtsverhältnis 1 Ag/P zu 1 Cu/P für Gesamtschicht c) verwendet werden. Entsprechend sind für b) auch Gemische TiO+Quarz, vorzugsweise im Gewichtsverhältnis 1 TiO zu 1 Quarz, verwendbar. Das Ausgangsgemisch aus Methanoldampf und Sauerstoff bzw. Luft und gegebenenfalls Wasserdampf und Inertgas wird in allgemeinen von oben nach unten geführt, so daß die obere Schicht (obere Schichten) a) gleichzeitig den dem Ausgangsgemisch vor der Reaktion zugewandten Teil bedeutet. Bei Reaktoren anderer Bauart oder anderer Führung des Ausgangsgemischs gelten sinngemäß alle Angaben der Beschreibung über oberen (unteren) Teil des Katalysators für den entsprechenden, dem Ausgangsgemisch vor der Reaktion (dem abgeführten Reaktionsgemisch) zugewandten Teil, z.B. bei horizontal angeordneten Reaktoren für den vorderen (hinteren) Teil des Katalysators. Im unteren Teil c) befinden sich 20 bis 94, vorzugsweise 40 bis 79 Gew.% aller Katalysatorteilchen, im mittleren Teil b) 1 bis 35, vorzugsweise 1 bis 20 Gew.% aller Katalysatorteilchen, im oberen Teil a) 5 bis 45, vorzugsweise 20 bis 40 Gew.% aller Katalysatorteilchen. Die Teilchen des unteren Schichtteils c) haben Korngrößen von 0,75 bis 2,5, die des mittleren Schichtteils b) von 0,1 bis 2,5, die des oberen Schichtteils a) 0,2 bis 1,75 Millimeter. Jeder Schichtteil kann aus einer oder mehreren Schichten, vorzugsweise aus 1 oder 2 Schichten bestehen. Bevorzugt ist ein 3- bis 9-Schichtenkatalysator, insbesondere ein 4- oder 5-Schichtenkatalysator. Jede dieser Schichten derselben Katalysatorkomponenten unterscheidet sich von der anderen derselben Katalysatorkomponente in der Korngröße der Teilchen und gegebenenfalls im zugehörigen Gewichtsanteil des Gesamtkatalysators.

Hat der obere Schichtteil a) 2 Schichten, so haben seine untere Schicht a2) bevorzugt einen Anteil von 2,5 bis 22,5 Gew.% und Teilchen einer Korngröße von 0,4 bis 1,75 Millimeter und seine obere Schicht a1) entsprechend einen Gewichtsanteil von 2,5 bis 22,5 Gew.% und Teilchen der Korngröße von 0,2 bis 1,0 Millimeter. Entsprechend sind bei dem mittleren Schichtteil b) mit Bezug auf Gewichtsanteil (Korngröße der Teilchen) bevorzugt:

b1) obere Schicht 0,5 bis 17,5 (0,1 bis 1,25 mm) Gew.%;
b2) untere Schicht 0,5 bis 17,5 (1,25 bis 2,5 mm) Gew.%.

Bei dem unteren Schichtteil c) sind bevorzugt:

c1) obere Schicht 10 bis 47 (0,75 bis 1,5 mm) Gew.%;
c2) untere Schicht 10 bis 47 (1,5 bis 2,5 mm) Gew.%.

Bezüglich Korngröße kann jede Schicht Teilchen des gesamten vorgennanten Korngrößenbereichs oder nur bestimmter Korngrößen innerhalb dieses Bereichs enthalten.

Die Schichtung jeder einzelnen Schicht ist meist regelmäßig, so daß die Schichtdicke der Einzelschicht über den ganzen Schichtquerschnitt hinweg gleich ist. In diesen Fällen hängt die Schichtdicke direkt von den vorgennanten Gewichtsanteilen Gesamtkatalysator und der jeweiligen Korngröße der Teilchen ab. Man kann aber auch eine unregelmäßige Schichtung aller oder mehrerer oder zweckmäßig einer Schicht vornehmen, z.B. in der Mitte, auf den Seiten oder vorteilhaft am Rande der Schicht die Hauptmenge der Katalysatorteilchen aufgeben und entsprechend nur eine kleinere Restmenge auf die übrige Schicht verteilen. In einer Auführungsform werden mehrere Einzelschichten oder vorteilhaft eine Einzelschicht nur am Rande der Katalysatorzone in Gestalt einer ring- oder kranzförmigen Schicht mit ebener Ober- und Unterseite der Schicht (Ringschicht) auf die jeweils darunterliegende, regelmäßig angeordnete Schicht mit regelmäßiger Schichtdicke aufgegeben. Da als Reaktoren üblicherweise ein Reaktionsrohr bzw. rohrförmige Reaktionsräume verwendet werden, liegt ein solcher Rand am äußeren Rohrkranz des Katalysatorträgers bzw. an der inneren Rohrwand.

Schicht c) enthält die Ag/P und/oder Cu/P-Teilchen. In der Regel werden die Teilchen durch Behandlung (Beizen) der Oberfläche der Metallteilchen mit Phosphorsäure, im allgemeinen o-Phosphorsäure, hergestellt. Zweckmäßig wird die Behandlung bei 10 bis 35°C während 1 bis 5 Stunden mit 0,1 bis 100, insbesondere 0,5 bis 10 Gramm o-Phosphorsäure je Kilogramm Ag und/oder Cu durchgeführt. Vorteilhaft werden 0,1- bis 5-gewichtsprozentige, wäßrige Phosphorsäurelösungen verwendet. Die Teilchen enthalten 99 bis 99,9999, vorteilhaft 99,95 bis 99,9995 Gewichtsprozent Kupfer und/oder Silber und 0,0001 bis 1, vorteilhaft 0,0005 bis 0,05 Gewichtsprozent Phosphor, bezogen auf das Gewicht dieser Teilchen an diesen Elementen. Die vorgennanten Angaben beziehen sich bezüglich Phosphor auf das Gewicht an elementarem Phosphor, unabhängig von der tatsächlichen Struktur als Element oder Phosphorverbindung, z.B. Silberphosphid, Kupferphosphid bzw. Silberphosphat, Kupferphosphat. Der Phosphor als Element oder Verbindung befindet sich auf und in der Oberfläche der Metallteilchen.

4

In der Regel belastet man den Katalysator mit 1 bis 3 t, insbesondere 1,4 bis 2,4 t Methanol je m² Katalysatorbettquerschnitt und Stunde. Zur großtechnischen Ausführung verwendet man bevorzugt Katalysatorbettdurchmesser von mindestens 0,5, zweckmäßig 1 bis 3 Meter.

Die Oxidation wird im übrigen in bekannter Weise durchgeführt, indem man z.B. ein Gasgemisch aus Methanoldampf, Luft, gegebenenfalls Inertgas und zweckmäßig Wasserdampf in vorgenannten Mengen bei in der Regel Temperaturen von 600 bis 750°C, insbesondere 600 bis 710°C, durch den Silberkatalysator leitet. Das Verfahren wird zweckmäßig bei Drükken zwischen 0,5 und 2 bar, vorzugsweise zwischen 0,8 und 1,8 bar, kontinuierlich durchgeführt. Es ist dabei vorteilhaft, die die Katalysatorzone verlassenden Reaktionsgase innerhalb kurzer Zeit abzukühlen, z.B. auf Temperaturen von 50 bis 350°C. Das abgekühlte Gasgemisch wird dann zweckmäßig einem Absorptionsturm zugeführt, in welchem der Formaldehyd mit Wasser, vorteilhaft im Gegenstrom, aus dem Gasgemisch gewaschen wird.

Der nach dem Verfahren der Erfindung herstellbare Formaldehyd ist Desinfektionsmittel, Gerbstoff, Reduktionsmittel und wertvoller Ausgangsstoff für die Herstellung von Kunstharzen, Klebmitteln und Kunststoffen. Bezüglich der Verwendung wird auf den genannten Band von Ullmann, Seite 670, verwiesen.

Beispiel 1

Man verwendete eine Anlage mit Methanol-Verdampfer (Bodenkolonne) und einem senkrechten Rohrreaktor. Der Reaktor enthielt an seinem Kopf die Zuführung für das dampfförmige Ausgangsgemisch vor der Reaktion und die Reaktorhaube. Die Katalysatorschicht lag unterhalb des Reaktorkopfes, weiter unten folgte eine Kühlzone. Der Reaktor war mit einer Absorptionskolonne verbunden.

Für die Katalysatorkomponenten in Schicht c wurde reines Silber 3 Stunden lang in einer 0,2-prozentigen $H_3PO_4$-Lösung bei 20°C gebeizt und anschließend getrocknet.

In den Reaktor wurde ein Katalysator (14 g) folgender Zusammensetzung eingetragen:

|  | Katalysator-komponente | Anteil am Katalysator (Gew.%) | Korngröße mm |
|---|---|---|---|
| Schicht a oben | Ag | 25 | 0,2—0,75 |
| Schicht b mitte | Quarz | 5,5 | 0,2—0,4 |
| Schicht c unten | Ag/P | 69,5 | 0,75—2,5 |

Der Durchmesser des Katalysators war 2 cm. Dem Verdampfer wurde pro Stunde ein Ausgangsgemisch von 470 g Methanol in Gestalt von Rohmethanol mit 1,5 Gew.% Verunreinigungen, 314 g Wasser und 944 g Luft zugeführt und verdampft. Die Luft wurde in den Verdampfersumpf eingeleitet und durch die Flüssigkeitsschichten (Methanol/Wasser) im Sumpf und in den Kolonnenböden des Verdampfers geführt. Das dampfförmige Ausgangsgemisch wurde durch den Katalysator geleitet und bei 700°C und 1,2 bar umgesetzt. Das Reaktionsgemisch wurde nun auf 150°C abgekühlt und in Wasser gelöst. In Form einer 40,2-gewichtsprozentigen Formaldehydlösung erhielt man 397 g pro Stunde Formaldehyd (ber. 100%), entsprechend einer Ausbeute (bezogen auf eingesetztes Methanol) von 90,1% der Theorie. Die Lebensdauer des Katalysators betrug 110 Tage. Die Formaldehydlösung hatte einen Gehalt von 2,4 Gew.% Methanol und 0,017 Gew.% Ameisensäure, bezogen auf Formaldehyd (ber. 100%). Der Umsatz betrug 98%. Die Selektivität (Ausbeute, bezogen auf umgesetztes Methanol) betrug 92%.

Beispiel 2

Es wurde dei gleiche Anlage wie in Beispiel 1 verwendet.

In den Reaktor wurde ein Katalysator (14 g) folgender Zusamnensetzung eingetragen:

|  | Katalysator-komponente | Anteil am Katalysator (Gew.%) | Korngröße mm |
|---|---|---|---|
| Schicht a oben | Ag | 45 | 0,2—0,75 |
| Schicht b mitte | TiO | 12 | 0,75—1 |
| Schicht c unten | Ag/P | 43 | 0,75—1 |

Die Umsetzung wurde analog Beispiel 1 durchgeführt, jedoch wurde statt Rohmethanol reines Methanol verwendet. In Form einer 40,3-gewichtsprozentigen Formaldehydlösung erhielt man 394,4 g pro Stunde Formaldhyd (ber. 100%), entsprechend einer Ausbeute (bezogen auf eingesetztes Methanol) von 89,5% der Theorie. Die Lebensdauer des Katalysators betrug 113 Tage. Die Formaldehydlösung hatte einen Gehalt von 3,8 Gew.% Methanol und 0,006 Gew.% Ameisensäure, bezogen auf Formaldehyd (ber. 100%). Der Umsatz betrug 96,8%, die Selektivität (Ausbeute, bezogen auf umgesetztes Methanol) betrug 92,5%.

5

Beispiel 3

Es wurde die gleiche Anlage wie in Beispiel 1 verwendet.

In den Reaktor wurde ein Katalysator (14 g) folgender Zusammensetzung eingetragen:

|  | Katalysator-komponente | Anteil am Katalysator (Gew.%) | Korngröße mm |
|---|---|---|---|
| Schicht a oben | Ag | 35 | 0,2—0,75 |
| Schicht b mitte | Quarz | 10 | 0,4—1 |
| Schicht c unten | Cu/P | 55 | 0,75—2,5 |

Die Umsetzung wurde analog Beispiel 1 durchgeführt, jedoch wurde statt Rohmethanol reines Methanol verwendet. In Form einer 40,8-gewichtsprozentigen Formaldehydlösung erhielt man 397 g pro Stunde Formaldehyd (ber. 100%), entsprechend einer Ausbeute (bezogen auf eingesetztes Methanol) von 90,1% der Theorie. Die Lebensdauer des Katalysators betrug 113 Tage. Die Formaldehydlösung hatte einen Gehalt von 3,3 Gew.% Methanol und 0,0065 Gew.% Ameisensäure, bezogen auf Formaldehyd (ber. 100%). Der Umsatz betrug 97,2%, die Selektivität (Ausbeute, bezogen auf umgesetztes Methanol) betrug 92,7%.

**Patentanspruch**

Verfahren zur Herstellung von Formaldehyd durch oxidierende Dehydrierung von Methanol in Gegenwart eines Silberkatalysators mit mehreren Schichten von Katalysatorteilchen bei erhöhter Temperatur, dadurch gekennzeichnet, daß die Umsetzung mit einem Katalysator mit 3 oder mehr Schichten Katalysatorteilchen und einer Gesamtschichtdicke von 5 bis 50 mm durchgeführt wird, wobei

a) ein Teil der Schichten 5 bis 45 Gew.% des Katalysators mit Silberkristallen der Korngröße 0,2 bis 1,75 Millimeter,

b) ein Teil der Schichten 1 bis 35 Gew.% des Katalysators mit Titanmonooxid- und/oder Quarzteilchen der Korngröße 0,1 bis 2,5 Millimeter und

c) der restliche Teil der Schichten 20 bis 94 Gew.% des Katalysators mit Teilchen von 99 bis 99,9999 Gew.% Kupfer und/oder Silber und 0,0001 bis 1 Gew.% Phosphor, bezogen auf das Gewicht dieser Teilchen an diesen Elementen, und der Korngröße 0,75 bis 2,5 Millimeter enthalten.

**Revendication**

Procédé de préparation de formaldéhyde par une déshydratation oxydante du méthanol à température accrue sur un catalyseur à l'argent constitué de plusieurs couches de particules du catalyseur, caractérisé en ce que la réaction est réalisée avec un catalyseur comportant trois ou plus de trois couches de particules du catalyseur, l'épaisseur totale des couches étant comprise entre 5 et 50 mm et

a) une partie des couches comprend 5 à 45% en poids du catalyseur et contient des cristaux d'argent avec des dimensions de 0,2 à 1,75 mm,

b) une autre partie des couches comprend 1 à 35% en poids du catalyseur et contient des particules de monoxyde de titane et(ou) de quartz d'une granulométrie de 0,1 à 2,5 mm et

c) la partie restante des couches constitue 20 à 94% en poids du catalyseur et contient des particules d'une teneur en cuivre et(ou) en argent de 99 à 99,9999% en poids et d'une teneur en phosphore de 0,0001 à 1% en poids, par rapport au poids de ces éléments dans ces particules, et d'une granulométrie de 0,75 à 2,5 mm.

**Claim**

A process for the preparation of formaldehyde by oxidative dehydrogenation of methanol in the presence of a silver catalyst, having a plurality of layers of catalyst particles, at elevated temperatures, wherein the reaction is carried out using a catalyst having three or more layers of catalyst particles and a total layer thickness of from 5 to 50 mm,

a) some of the layers containing from 5 to 45% by weight of the catalyst and comprising silver crystals having a particle size of from 0.2 to 1.75 millimeter,

b) some of the layers containing from 1 to 35% by weight of the catalyst and comprising titanium monoxide and/or quartz particles having a particle size of from 0.1 to 2.5 millimeter and

c) the remainder of the layers containing from 20 to 94% by weight of the catalyst and comprising particles consisting of from 99 to 99.9999% by weight of copper and/or silver and from 0.0001 to 1% by weight of phosphorus, the percentages being based on the weight of these elements in these particles, and having a particle size of from 0.75 to 2.5 millimeter.